# EUROPEAN PATENT APPLICATION

(11) **EP 4 159 055 A1**
(43) Date of publication of application: **05.04.2023**
(21) Application number: 21200162.2
(22) Date of filing: 30.09.2021
(51) Int. Cl.: A24F 40/10, A24F 40/30, A24F 42/20, A61M 15/00

(54) **SMOKING SUBSTITUTE APPARATUS**

(71) Applicant: NERUDIA LIMITED, Liverpool Merseyside L24 9HP (GB)
(72) Inventor: STUART, Ian, Liverpool, 24 9HP (GB); MAXWELL-HOGG, Steven, Liverpool, 24 9HP (GB); MURRAY, Samantha, Liverpool, 24 9HP (GB)
(74) Representative: Mewburn Ellis LLP

(57) **Abstract**

A smoking substitute apparatus is provided which includes a compartment comprising an aerosol former, a wick downstream of the compartment, a cavity adjacent to the wick, adapted to receive and hold a frangible capsule comprising a property modifying agent, and a cap. The cap is movable between a preactivated position in which the cavity is adapted to hold an intact frangible capsule, and an activated position in which an impingement surface of the apparatus intrudes into the cavity. The apparatus finds use in a smoking substitute system which can deliver flavour to a user.

## Description

### Field of the Invention

The present invention relates to a smoking substitute apparatus and particularly, although not exclusively, to a smoking substitute apparatus that is able to deliver flavour to a user whilst keeping the flavourant and aerosol former separate.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful byproducts. There have been proposed various smoking substitute devices (or "substitute smoking devices") in order to avoid the smoking of tobacco.

Such smoking substitute devices can form part of nicotine replacement therapies aimed at people who wish to stop smoking and overcome a dependence on nicotine.

Smoking substitute devices may comprise electronic systems that permit a user to simulate the act of smoking by producing an aerosol, also referred to as a "vapour" that is drawn into the lungs through the mouth (inhaled) and then exhaled. The inhaled aerosol typically bears nicotine and/or flavourings without, or with fewer of, the odour and health risks associated with traditional smoking.

In general, smoking substitute devices are intended to provide a substitute for the rituals of smoking, whilst providing the user with a similar experience and satisfaction to those experienced with traditional smoking and with combustible tobacco products.

The use of smoking substitute systems has grown rapidly in the past few years as an aid to assist habitual smokers wishing to quit tobacco smoking. There are a number of different categories of substitute smoking systems, each utilising a smoking substitute approach.

Some smoking substitute devices are designed to resemble a traditional cigarette and are cylindrical in form with a mouthpiece at one end. Other smoking substitute devices do not generally resemble a cigarette (for example, the smoking substitute device may have a generally box-like form).

There are a number of different categories of smoking substitute devices, each utilising a different smoking substitute approach. A smoking substitute approach corresponds to the manner in which the substitute system operates for a user.

One approach for a smoking substitute device is the so-called "vaping" approach, in which a vaporisable liquid, typically referred to (and referred to herein) as "e-liquid", is heated by a heating device to produce an aerosol vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices, which typically have a sealed tank and heating element. The tank is pre-filled with e-liquid and is not intended to be refilled by an end user. One subset of closed system vaping smoking substitute devices include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute devices are completely disposable, and intended for one-use only.

There are also "open system" vaping smoking substitute devices which typically have a tank that is configured to be refilled by a user. In this way the device can be used multiple times.

An example vaping smoking substitute device is the myblu^{™} e-cigarette. The myblu^{™} e-cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

As explained above, in the so-called "vaping" approach, e-liquid is heated by a heating device to produce an aerosol vapour which is inhaled by a user. Many e-cigarettes also deliver flavour to the user to enhance the experience. In such e-cigarettes, e-liquid is often sold as a flavoured product, e.g. a specific blend of flavour compounds are already homogeneously mixed with the e-liquid during the manufacturing process. As such, the user would have to purchase flavoured consumables available on the market with limited opportunities to personalise the vaping experience according to their preferences. Further, when blended in the e-liquid, flavour components may interact with other constituents in the e-liquid during storage. In addition, it may put additional strain on the supply chain for distributing a large variety of consumables each having different flavours and strengths.

There may be a need for improved design of smoking substitute systems, in particular in regards to the delivery of flavour to a user.

The present invention has been devised in light of the above considerations.

### Summary of the Invention

At its most general, the present invention relates to a smoking substitute apparatus having a separate storage for aerosol former and a capsule which may contain a property modifying agent and also having means to release the property modifying agent while keeping it separate from the aerosol former. This allows the user to experience modified properties when using the apparatus without having to store the apparatus with the property modifying agent and the aerosol former mixed. Further, it may allow the user to specify the quantity and type of flavour or other property modifying agent to be added to the aerosol former. Thus, it may enable the user to obtain a specific flavour or other property tailored to the user's needs. The separation of the aerosol former and the property modifying agent is beneficial to manufacturing processes, as larger volumes of unmodified (e.g. unflavoured) e-liquid can be produced and distributed, rather than modification being required during manufacture. This also reduces plant downtime as filling equipment no longer needs to be purged and cleaned between filling to prevent cross-contamination of differently modified aerosol formers.

Furthermore, the separate provision of a property modifying agent improves the quality of the vapour produced. When property modifying agent is included within the aerosol former, the exposure to heat during the vaporisation process causes thermal degradation of compounds in the property modifying agent (e.g. flavour compounds), which leads to increased level of toxins in the vapour. By instead providing an unmodified aerosol former and adding property modifying agent after vaporisation and at a downstream location away from the heating element, such effects of thermal degradation of the property modifying agent are reduced or eliminated.

According to a first aspect of the present invention, there is provided a smoking substitute apparatus, comprising:
a compartment comprising an aerosol former,
a wick downstream of the compartment,
a cavity adjacent to the wick, adapted to receive and hold a frangible capsule comprising a property modifying agent, and
a cap, wherein
the cap is movable between a pre-activated position in which the cavity is adapted to hold an intact frangible capsule, and an activated position in which an impingement surface of the apparatus intrudes into the cavity;
such that, when a frangible capsule is positioned within the cavity, movement of the cap from the pre-activated position to the activated position ruptures the capsule to release the property modifying agent to the wick.

The aerosol former may be an e-liquid. The e-liquid may, for example, comprise a base liquid and nicotine. The base liquid may include propylene glycol and/or vegetable glycerine. The e-liquid may be flavourless. That is, the e-liquid may not contain any flavourant and may consist solely of a base liquid of propylene glycol and/or vegetable glycerine and nicotine.

The property modifying agent may comprise a flavourant. The flavourant may be provided in solid, gel or liquid form. It may include menthol, liquorice, chocolate, fruit flavour (including e.g. citrus, cherry etc.), vanilla, spice (e.g. ginger, cinnamon) and tobacco flavour. The flavourant may modify a flavour of the aerosol former upon contacting or mixing with the aerosol former. The property modifying agent may also be water which may provide improved flavour delivery to the user.

The frangible capsules may allow the property modifying agent to be stored therein, and therefore be kept separate from the aerosol former stored in the first compartment during transportation and storage. Once the property modifying agent is released, it is absorbed by the wick. Since the wick is downstream of the aerosol former, the property modifying agent is kept away from the aerosol former and any heating element which may degrade the property modifying agent, even after release of the property modifying agent. During inhalation, vapour is drawn up from the body containing the aerosol former, passing through the wick material where it is modified by the property modifying agent, for example to impart flavour.

The wick may be of any known porous fibre or polymer construction, such as cellulose acetate or polyethylene foam. The wick material is sized suitably to ensure at least all the property modifying agent can be absorbed without the wick becoming saturated. The wick allows vapour to be drawn through from the aerosol former by inhalation of the user. The wick material can be formed in a variety of shapes and may contain geometry to help encourage flavour transfer or reduce total particulate matter (TPM) loss. The wick may have a central hollow bore or the wick may have multiple bores. One or multiple hollow bores would reduce TPM loss but not reduce flavour delivery to the user. The wick material may also prevent any parts of the shell of the capsule shell from being inhaled by the user.

The wick may be pre-imbued with a property modifying agent prior to insertion into the wick recess of the cap. For example, the wick may be pre-imbued with a flavourant. This enables delivery of the property-modifying agent form the capsule alongside the property modifying agent already imbued in the wick.

In some embodiments, the cavity is defined between the cap and a base of the apparatus to which the cap is fitted, wherein the base and the cap are movable relative to one another causing the impingement surface to intrude into the cavity. In some embodiments, the impingement surface forms a part of the base of the apparatus, and moving the cap towards the base causes the impingement surface to intrude into the cavity.

In some embodiments the cap and the base are separate structures associated with one another in a friction fit arrangement. In some embodiments the friction fit allows for sliding of the cap relative to the base between the pre-activated position and the activated position. In some embodiments the base and the cap comprise a detent mechanism to temporarily hold the apparatus in the pre-activated position until the user activates the apparatus to move it into the activated position. The detent mechanism may comprise a tab on one of the base and the cap, and a complementary recess on the other of the base and the cap, to receive the tab. The tab may comprise an inclined surface to facilitate sliding of the apparatus into the activated position whilst preventing movement back into the pre-activated position.

Conveniently, the cap may be moveable between the pre-activated position and the activated position by a ratchet mechanism. This provides discrete separation of the two positions and the user may feel or hear a click that informs them the activated position has been reached.

In some embodiments the cavity adjacent to the wick, adapted to receive and hold a frangible capsule comprising a property modifying agent, comprises a recess within the cap. In other words, the cap defines a recess which forms at least part of the cavity and is adapted to receive at least part of the capsule. In some embodiments the cap is a structure having a generally concave shape and the recess is located within an internal wall of the cap, such that when the concave cap having the recess is pushed onto a base having a complementary convex structure, the capsule sits in a location between the internal wall of the cap and an external portion of the base. In this way, pushing the cap further into place towards the base may move the cap into the activated position to rupture the capsule.

The recess may form a portion of the cavity, with the remainder of the cavity which is adapted to hold the capsule being defined by an empty internal volume between the cap and the base.

In some embodiments, the impingement surface is a surface of a structure of the base of the apparatus onto which the cap is fitted. The structure may be a protrusion which, in the activated position of the cap, is adapted to extend into at least a portion of the volume occupied by the capsule when the capsule is held within the cavity.

In some embodiments, the impingement surface comprises a planar surface lying in a plane which is perpendicular to the direction of movement of the impingement surface relative to the cap when the cap is moved from the pre-activated position into the activated position. In this way, the plane of the impingement surface contacts the capsule in a blunt manner, increasing the force imparted on the capsule and ensuring rupture of the capsule under the minimal applied force by the user.

The structure carrying the impingement surface need not extend into the recess itself when the cap is in the activated position, provided that the impingement surface extends into at least a portion of the volume occupied by the capsule when the capsule is held within the cavity. In other words, the capsule itself may protrude from the volume of the recess when held within the cavity, such that the capsule may be ruptured by the impingement surface without the need for the surface to enter the recess itself. In this way, it is not necessary that the impingement surface have a shape, size or location to facilitate entry into the recess, provided that it is adapted to contact and put pressure on the capsule in the activated position.

The recess may be dimensioned to facilitate holding of the capsule in a fixed position. For example, for a spherical capsule, the recess may receive a portion of the capsule thereby providing a seat for the capsule to prevent the capsule "rolling" out of its position within the cavity.

In some embodiments, the recess within the inner wall of the cap is adapted to hold the capsule in position after the capsule has been received by the recess. In other words, once received by the recess the capsule may remain fixed in position within the recess when subjected to e.g. normal gravitational forces. This may be achieved by a friction fit between the recess and the capsule, for example by tailoring the capsule diameter.

However, such fixing of the capsule within the recess itself is not necessary, provided that the capsule remains located in the cavity when the cap is in the pre-activated position. For example, the capsule may be held in position between the surface of the cap on one side and a surface of the base of the apparatus on the other side, such that the capsule is "sandwiched" in place prior to activation and rupture. The capsule may be held in position between the surface of the cap on one side and the impingement surface of the apparatus on the other side.

Optionally, the cavity may be adapted to receive and hold more than one frangible capsule. This enables the user to imbue the wick with multiple property modifying agents, potentially mixing and matching different properties or amplifying the effects of one property modifying agent.

Conveniently, the apparatus may comprise a plurality of cavities adjacent to the to the wick, wherein each cavity is adapted to receive and hold a frangible capsule comprising a property modifying agent. This enables the user to use multiple property modifying agents to alter the properties of the aerosol former. For example, the user may use two capsules containing the same flavourant for a more intense flavour, or the user may use a first capsule containing a first flavourant and a second capsule comprising a second flavourant, wherein the first and second flavourants are different, to mix flavours as desired.

Advantageously, the cap may be moveable to a second activation position wherein the impingement surface of the apparatus advances further towards the cavity than in the activated position, such that when more than one frangible capsule is positioned with the same or different cavity, movement of the cap from the pre-activated position to the activated position ruptures a first capsule to release the property modifying agent to the wick and movement from the activated position to the second activated position ruptures a second capsule to release the property modifying agent to the wick. Using this mechanism, the user may rupture the frangible capsules at separate times, for example when a different flavour is desired, or a more intense flavour is desired.

Advantageously, the cavities may be arranged regularly around the wick. This ensures that when the property modifying agent is released from the capsules, it is released evenly around the wick and will be easily be distributed evenly throughout the wick. For example, the apparatus may have two cavities on opposite sides of the wick.

Optionally, each cavity may have the same volume as each other in the activated position. This may enable the capsules to be ruptured at the same time with equal force of the impingement surface. The multiple cavities may be of identical dimensions, such that (provided that the capsules used in each cavity are the same size) the capsules will rupture at the same time.

Optionally, the cavities may have different dimensions compared to each other in the activated position. This may enable one frangible capsule to be ruptured and the property modifying agent released to the wick without rupturing a second frangible capsule. Therefore, the capsules can be ruptured at different times to each other and require different application of force from the impingement surface. This provides the user with more control over the properties of the aerosol.

In some embodiments, the cavity when the cap is in the pre-activated position defines an empty volume which is capable of receiving a sphere having a diameter of from 0.2 to 5.0 mm, for example from 0.5 to 5.0 mm. In some embodiments, the maximum diameter of a sphere which is capable of being received by the cavity when the cap is in the pre-activated position is from 0.2 to 5.0 mm, for example from 0.5 to 5.0 mm. In this way, the apparatus is able to hold a frangible capsule having a diameter within this range, which has been found to be suitable for delivering an appropriate volume of property modifying agent to the wick.

In some embodiments, the maximum diameter of a sphere which is capable of being received by the cavity when the cap is in the pre-activated position is D₁ and the maximum diameter of the sphere which is capable of being received by the cavity when the cap is in the activated position is D₂, wherein D₂ < D₁. This ensures that a capsule of diameter D₁ held within the cavity in the pre-activated position is ruptured by the impingement surface in the activated position. In some embodiments, (D₁ - D₂) is from 0.1 to 3 mm, for example from 0.5 to 2 mm. In some embodiments, D₁/D₂ is from 1.2 to 1.8, for example from 1.4 to 1.7.

In some embodiments, the apparatus comprises a first cavity and a second cavity, wherein the maximum diameter of a sphere which is capable of being received by the first cavity when the cap is in the pre-activated position is D₁ and the maximum diameter of the sphere which is capable of being received by the first cavity when the cap is in the activated position is D₂, and wherein the maximum diameter of a sphere which is capable of being received by the second cavity when the cap is in the pre-activated position is D₃ and the maximum diameter of the sphere which is capable of being received by the second cavity when the cap is in the activated position is D₄, wherein D₂ < D₁ and D₄ < D₃. In some embodiments, D₁ = D₃. In some embodiments, D₂ = D₄. In some embodiments, D₁ = D₃ and D₂ = D₄.

Advantageously, the apparatus may comprise a frangible capsule containing a flavourant in one cavity and a frangible capsule containing water in another cavity. This may improve flavour delivery. Water may allow for a quicker rate of absorption of the flavour compounds to the wick and ultimately the user. Water in this situation may act as a solvent and facilitate an increased and more efficient dissolution rate to the wick and thus allow for more flavour to be delivered to the user.

The smoking substitute apparatus of the first aspect does not contain the capsule but is adapted to receive the capsule. The apparatus may be supplied to the user without any capsule in place and the user is at liberty to independently obtain capsules having the desired property modifying agent (e.g. flavourant) and add them to the apparatus before use, for example by removing the cap and inserting the capsule into the cavity before replacing the cap and "activating" the apparatus. In this way the user may have complete control over the experience.

However the capsule may be added to the apparatus during manufacture and supplied to the user in the pre-activated position, such that the user simply "activates" the apparatus by moving the cap into the activated position to release the property modifying agent if desired before use.

Therefore according to a second aspect of the present invention, there is provided a smoking substitute apparatus, comprising:
a compartment comprising an aerosol former,
a wick downstream of the compartment,
a cavity adjacent to the wick,
a frangible capsule comprising a property modifying agent, the frangible capsule being located within the cavity, and
a cap, wherein
the cap is movable between a pre-activated position in which the cavity holds the intact frangible capsule, and an activated position in which an impingement surface of the apparatus is advanced further towards the cavity than in the pre-activated position;
such that movement of the cap from the pre-activated position to the activated position ruptures the capsule to release the property modifying agent to the wick.

The apparatus of the second aspect comprises the apparatus of the first aspect, along with a capsule in position within the cavity.

The invention includes the combination of the aspects and preferred features described except where such a combination is clearly impermissible or expressly avoided.

Suitable frangible capsules may have a diameter of 0.2 to 5.0 mm, for example 0.5 to 5.0 mm. This corresponds to a volume of 0.0000042 to 0.0090 ml of property modifying agent which may be encapsulated in the capsule. Therefore, the cavity may have dimensions of 0.2 to 6.0 mm in the preactivated position in order to accommodate the frangible capsule.

The frangible capsules may be ruptured under a force of 5 to 50 kg·m/s². Preferably the frangible capsules may be ruptured under a force of 10 to 20 kg·m/s². Therefore, the impingement surface may be capable of exerting a force of 5 to 50 kg·m/s² on a capsule in the cavity. Preferably the impingement surface is capable of exerting a force of 10 to 20 kg·m/s² on a capsule in the cavity.

The frangible capsules may be formed using a microencapsulation technique which combines the basic processing techniques of coextrusion, vibrational nozzle technique and, UV curing.

The coextrusion technique is a favourable method to produce medium-to-large-size microcapsules with an inner core -outer shell -morphology. Two immiscible liquids are simultaneously extruded through concentric nozzles in order to produce a biliquid stream. Under the influence of gravitational, centrifugal or other forces, this bi-liquid stream separates into discrete droplets taking on inner core ― outer shell morphology.

The liquid outer shell is then made to undergo a physical or chemical change to harden the outer shell. This hardening may be affected by heating to remove a solvent or by cooling to solidify a molten shell material. This method provides a technically assisted clear separation of core and shell material. This method may be combined with vibrational nozzle technique. The vibrational nozzle technique is a well-established method for micro-granulation and matrix-type encapsulation. A laminar material stream is generated by means of a nozzle, which is subjected to sinusoidal vibrations. These vibrations induce constrictions of the stream leading to droplet separations. By proper frequency adjustment, very uniform droplets can be achieved. By using units up to several thousand nozzles, the process can easily be up scaled to industrial standards.

Capsule size is adjustable using this method. Monodispersity of capsules produced by the method may have a standard deviation of less than 2 %.

In some embodiments, capsules have a sphericity of at least 0.95, for example at least 0.96, at least 0.97, at least 0.98 or at least 0.99.

The outer shell of the frangible capsule may have a viscosity at processing in the range of about 0.001 to 10 N·s/m². The curing speed of the outer shell material, wherein the material is hardened, may be in the range of 0.01 to 0.1 s.

The outer shell material may be generated from monomeric components such as acrylate and methacrylate monomers or monomers comprising organic-inorganic hybrid material. Examples of monomeric materials suitable for generating the outer shell are shown in Table 1.

**Table 1: Monomeric Building Blocks for Shell Material.**

| **Name** | **Structure** |
|---|---|
| Trimethylolpropane triacrylate (TMPTA) | 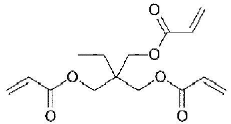 |
| Pentaerythritol triacrylate (PETIA) | 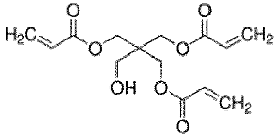 |
| Bisphenol A dimethacrylate (BPADMA) | 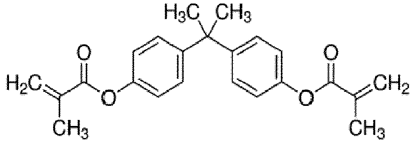 |
| Tricyclodecanedimethanol diacrylate (TCDDMDA) | 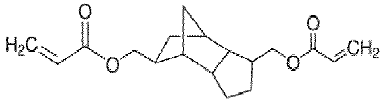 |
| Tris(2-Hydroxyethyl) isocyanurate-triacrylate (THEICTA) | 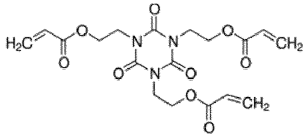 |
| Polyethylenglycol diacrylate | 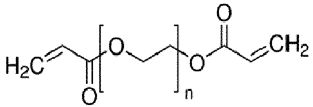 |
| Ethoxylated bisphenol A diacrylate (BPADAE) | 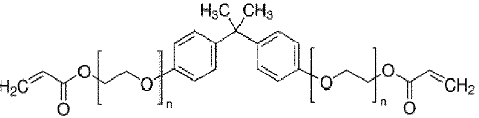 |
| Pentaerythritol Tetraacrylate (PETTA) | 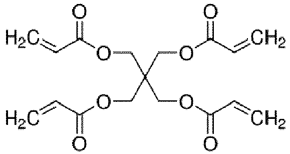 |
| Esterdiole-Diacrylate | 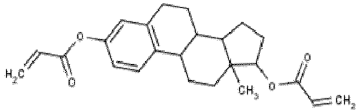 |
| Hexanediol Diacrylate | 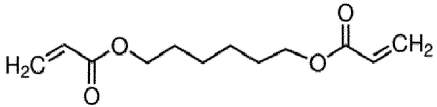 |
| Decanediol Diacrylate | 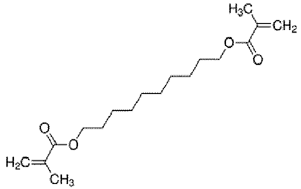 |
| Urethane Dimethacrylate (UDMA) | 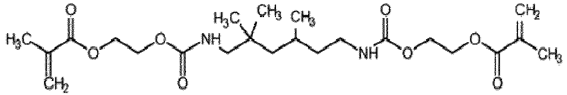 |

Photocurable materials may also be suitable for generating the outer shell material. By using a combination of a photo initiator, a monomer and an oligomer, capsules can be produced with varying parameters such as strength or wall thickness.

The properties of a photocured material, such as flexibility, adhesion, and chemical resistance may be provided by the functionalized oligomers present in the photocurable composite. The oligomer may be an epoxide, polyether, urethane, or polyester. In some instances, the oligomer is a polyurethane. In some instances, the oligomer is a polyether. In some instances, the oligomer is a polyester. Formulations for manufacturing capsules may comprise a mixture of several types of oligomers to achieve the desirable properties for a material, for example a combining any two or more of epoxide, polyether, urethane, or polyester.

The monomers used in radiation curable systems help control the speed of cure, crosslink density, final surface properties of the film, and viscosity of the resin. The monomers may be, for example, styrene, N-vinylpyrrolidone, and acrylates. Several different monomers may be used in the formulation. Examples of suitable monomers for photopolymerization are shown in Table 2.

**Table 2: Monomers for Photopolymerization**

| **Name** | **Structure** |
|---|---|
| Diethylene glycol divinyl ether (DEGDE) | 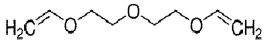 |
| 1,4-Cyclohexanedimethanol divinyl ether (CHDMDE) | 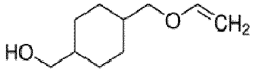 |
| Triethylene glycol divinyl ether (TEGDE) | 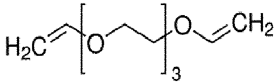 |
| Styrene | 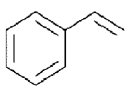 |
| N-vinyl pyrrolidone | 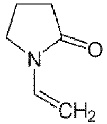 |

The photo initiator may be a free radical photo initiator. For example, the photo initiator may be a benzoin ether, benzal ketal, phosphine oxide, benzophenone derivative, thioxanthone derivative or 1,2-diketone. Specific examples of photo initiators are shown in Table 3.

**Table 3: Photo initiators for Photopolymerization**

| **Name** | **Structure** |
|---|---|
| Benzoin ethyl ether | 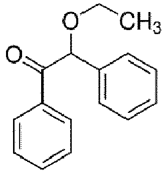 |
| 2, 2-dimethoxy-2-phenylacetophenone | 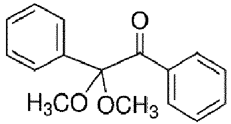 |
| Acyl phosphine oxides | 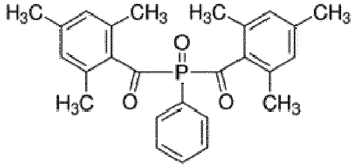 |
| Benzophenone | 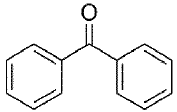 |
| 2-((9-oxo-9H-thioxanthen-2-yl) oxyl) acetic acid | 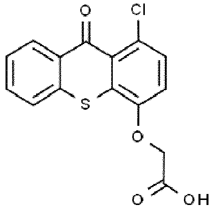 |
| Benzil | 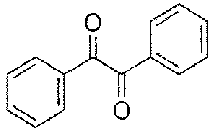 |

### Summary of the Figures

So that the invention may be understood, and so that further aspects and features thereof may be appreciated, embodiments illustrating the principles of the invention will now be discussed in further detail with reference to the accompanying figures, in which:
**Figure 1A** is a front view of a smoking substitute system, according to a first embodiment, in an engaged position.
**Figure 1B** is a front view of a smoking substitute system of the first embodiment in a disengaged position.
**Figure 1C** is a section view of a smoking substitute apparatus of the first embodiment.
**Figure 2A** is a perspective view of a cap according to a second embodiment.
**Figure 2B** is a perspective view of a cap according to a second embodiment showing capsules.
**Figure 3A** is a section view of a smoking substitute system according to a second embodiment in a pre-activated position.
**Figure 3B** is a section view of a smoking substitute system according to a second embodiment in an activated position.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

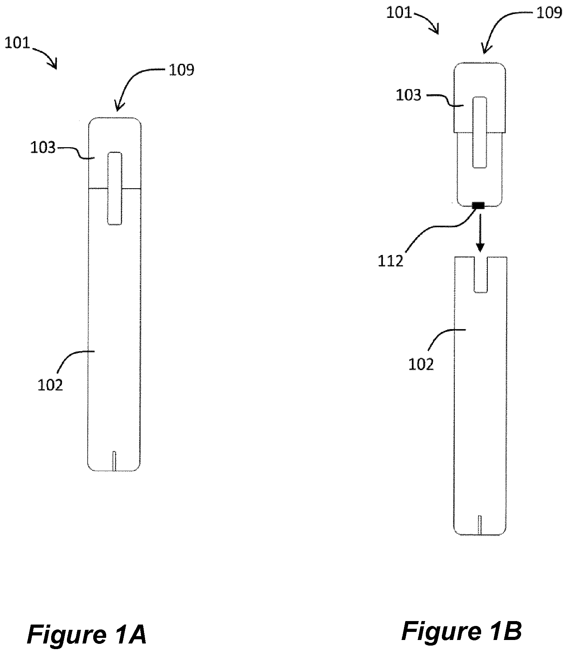

**Figures 1A** and **1B** illustrate a smoking substitute system in the form of an e-cigarette system 101. The system 101 comprises an e-cigarette device defining a main body 102 of the system 101, and an smoking substitute apparatus in the form of an e-cigarette consumable (or "pod") 103. The smoking substitute apparatus is a smoking substitute apparatus. In the illustrated embodiment the consumable 103 (smoking substitute apparatus) is removable from the main body (e-cigarette device), so as to be a replaceable component of the system 101. In other words, the e-cigarette system 101 is a closed system.

As is apparent from Figures 1A and 1B, the consumable 103 is configured to engage the main body 102. Figure 1A shows the main body 102 and the consumable 103 in an engaged state, whilst Figure 1B shows the main body 102 and the consumable 103 in a disengaged state. When engaged, a portion of the consumable 103 is received in a cavity of the main body 102 and is retained in the engaged position by way of a snap-engagement mechanism. In other embodiments, the main body 102 and consumable 103 may be engaged by screwing one into (or onto) the other, through a bayonet fitting, or by way of an interference fit.

The system 101 is configured to vaporise an aerosol-former, which in the illustrated embodiment, is in the form of a nicotine-based e-liquid 104. The e-liquid 104 comprises nicotine and a base liquid including propylene glycol and/or vegetable glycerine. In the present embodiment, the e-liquid 104 is flavourless (and does not include any added flavourant). That is, if the e-liquid 104 were to be inhaled (i.e. in aerosol form) by a user, it would not have a particularly perceptible flavour or taste.

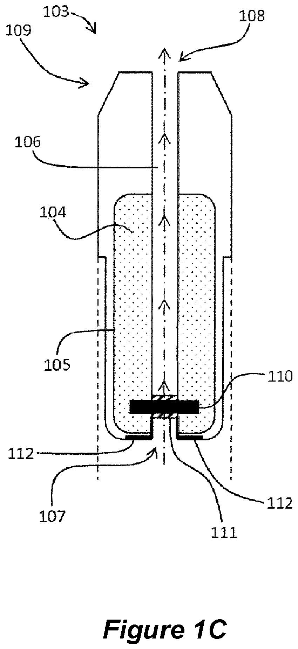

As is more apparent from **Figure 1C****,** this e-liquid 104 is stored within a reservoir in the form of a tank 105 that forms part of the consumable 103. In the illustrated embodiment, the consumable 103 is a "single-use" consumable 103. That is, upon exhausting the e-liquid 104 in the tank 105, the intention is that the user disposes of the entire consumable 103. In other embodiments, the e-liquid (i.e. aerosol former) may be the only part of the system that is truly "single-use". That is, the tank may be refillable with e-liquid or the e-liquid may be stored in a non-consumable component of the system. For example, the e-liquid may be stored in a tank located in the main body or stored in another component that is itself not single-use (e.g. a refillable cartomizer).

The tank 105 surrounds, and thus defines a portion of, a passage 106 that extends between an inlet 107 and an outlet 108 at opposing ends of the consumable 103. In this respect, the passage comprises an upstream end at the end of the consumable 103 that engages with the main body 102, and a downstream end at an opposing end of the consumable 103 that comprises a mouthpiece 109 of the system 101. When the consumable 103 is engaged with the main body 102, a user can inhale (i.e. take a puff) via the mouthpiece 109 so as to draw air through the passage 106, and so as to form an airflow (indicated by arrows) in a direction from the inlet 107 to the outlet 108 of the passage 106. Although not illustrated, the passage 106 may be partially defined by a tube (e.g. a metal tube) extending through the consumable 103. The passage 106 is in fluid communication with a gap defined between the consumable 103 and the main body 102 (when engaged) such that air outside of the system 101 is drawn into the passage 106 (during an inhale).

The smoking substitute system 101 is configured to vaporise the e-liquid 104 for inhalation by a user. To provide this, the consumable 103 comprises a heater having of a porous wick 110 and a resistive heating element in the form of a heating filament 111 that is helically wound around a portion of the porous wick 110. The porous wick 110 extends across the passage 106 (i.e. transverse to a longitudinal axis of the passage106) and opposing ends of the wick 110 extend into the tank 105 (so as to be submerged in the e-liquid 104). In this way, e-liquid 104 contained in the tank 105 is conveyed from the opposing ends of the porous wick 110 to a central portion of the porous wick 110 so as to be exposed to the airflow in the passage 106 (i.e. caused by a user inhaling).

The helical filament 111 is wound about this exposed central portion of the porous wick 110 and is electrically connected to an electrical interface in the form of electrical contacts 112 mounted at the end of the consumable that is proximate the main body 102 (when engaged). When the consumable 103 is engaged with the main body 102, the electrical contacts 112 contact corresponding electrical contacts (not shown) of the main body 102. The main body electrical contacts are electrically connected to a power source (not shown) of the main body 102, such that (in the engaged position) the filament 111 is electrically connected to the power source. In this way, power can be supplied by the main body 102 to the filament 111 in order to heat the filament 111. This heat is transferred from the filament 111 to the porous wick 110 which causes e-liquid 104 conveyed by the porous wick 110 to increase in temperature to a point at which it vaporises. The vaporised e-liquid becomes entrained in the airflow and, between the vaporisation point at the filament 111 and the outlet 108 of the passage 106, condenses to form an aerosol. This aerosol is then inhaled, via the mouthpiece 109, by a user of the system 101.

The power source of the main body 102 may be in the form of a battery (e.g. a rechargeable battery). The main body 102 may comprise a connector in the form of e.g. a USB port for recharging this battery. The main body 102 may also comprise a controller that controls the supply of power from the power source to the main body electrical contacts (and thus to the filament 111). That, is the controller may be configured to control a voltage applied across the main body electrical contacts, and thus the voltage applied across the filament 111. In this way, the filament 111 may only be heated under certain conditions (e.g. during a puff and/or only when the system is in an active state). In this respect, the main body 102 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 102 and consumable 103 may comprise a further interface which may, for example, be in the form of an RFID reader, a barcode or QR code reader. This interface may be able to identify a characteristic (e.g. a type) of a consumable 103 engaged with the main body 102. In this respect, the consumable 103 may include any one or more of an RFID chip, a barcode or QR code, or memory within which is an identifier and which can be interrogated via the interface.

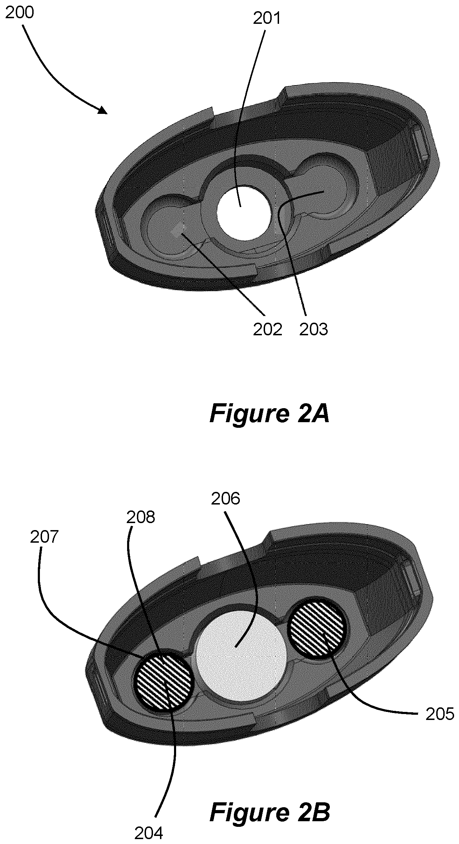

**Figure 2A** shows a cap 200 according to an embodiment of the present invention. The cap is generally dome-shaped and Figure 2A shows a perspective looking into the inner volume of the cap. The cap is separate from but connectable to the compartment of the apparatus comprising an aerosol former (not shown). The cap 200 defines a wick recess 201 for holding a wick (not shown) and two capsule recesses 202, 203, each for holding a frangible capsule. **Figure 2B** shows the cap 200 with a frangible capsule 204, 205 in each capsule recess 202, 203 and a wick 206 in the wick recess 201. Each capsule comprises an outer frangible shell 207 and a property modifying agent 208. The property modifying agent in each capsule may be the same or the property modifying agent in each capsule may be different. The property modifying agent may be water.

The capsule recesses 202, 203 are adjacent to the wick recess 201 and form part of the cavity when the cap is connected to the compartment comprising an aerosol former.

The capsule recesses 202, 203 are positioned uniformly around the wick recess 201. That is, the capsule recesses 202, 203 are positioned on opposite sides of the wick recess 201, located at the same distance from the wick recess 201.

The capsule recesses 202, 203 are at equal depth in the cap 200 such that the capsules 204, 205 protrude to an equivalent extent from the cap 200. This ensures that they rupture simultaneously when an impingement surface advances towards the capsules.

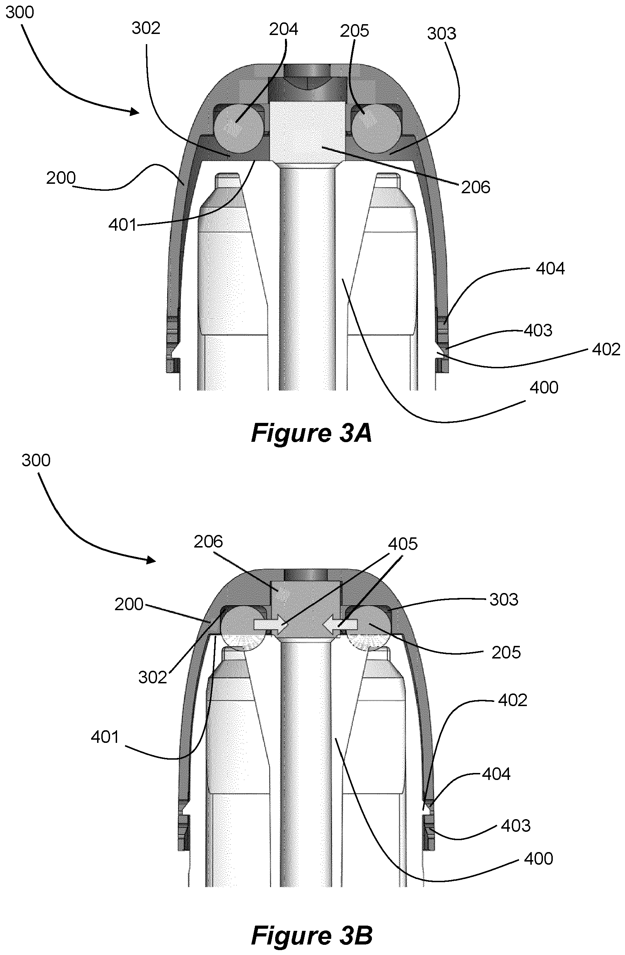

**Figure 3A** shows a smoking substitute apparatus 300 according to an embodiment of the present invention in a pre-activated position and **Figure 3B** shows a smoking substitute apparatus 300 according to an embodiment of the present invention in an activated position. The smoking substitute apparatus includes the cap 200 described above fitted onto a base portion 400. The base portion contains a heater and source of e-liquid (not shown) to form aerosol. The base portion 400 and cap 200 together form a consumable or "pod" equivalent to the consumable 103 shown in Figure 1. The consumable fits into the main body 102 to be used as described above. The smoking substitute apparatus (consumable) 300 has a wick 206 and two cavities 302, 303 adjacent to the wick. Each cavity 302, 303 is formed partially by the capsule recesses 202, 203 shown in **Figures 2A** and **2B** and partially by empty space defined between the cap 200 and the base portion 400 when the cap 200 is in the pre-activated position. The smoking substitute apparatus also has impingement surfaces 401. In the pre-activated position of **Figure 3A****,** the impingement surface does not intrude into the cavities 302, 303. Each cavity 302, 303 has the same volume.

The cap 200 is prevented from moving from the pre-activated position to the activated position by a detent mechanism. The detent mechanism comprises a tab 402 on the base and a first complementary recess 403 on the cap to receive the tab 402 and a second complimentary recess 404 on the cap. The tab 402 has an inclined surface to facilitate sliding of the apparatus into the activated position whilst preventing the movement back into the pre-activated position. Thus, the detent mechanism acts like a ratchet. This mechanism also fits the cap 200 to the base 400.

To release the property modifying agent the cap is moved from the preactivated position shown in **Figure 3A** to the activated position shown in **Figure 3B****.** The user applies force to the cap 200 and/or the compartment comprising an aerosol former (base) 400. The applied force moves the tab 402 of the detent mechanism to the second complimentary recess 404. As the cap 200 is moved to the activated position the impingement surface 401 intrudes into the cavities 302, 303 and the capsules 204, 205 become crushed against the inner surface of the cavities 302, 303, breaking the outer shells 207 of the capsules 204, 205 and releasing the property modifying agent 208. The property modifying agent 208 is then drawn into the wick 206 in the direction of arrows 405.

The impingement surface 401 in **Figures 3A** and **3B** is a flat surface. However, the impingement surface of the present invention may also be a pointed surface, configured to pierce the frangible capsules 204, 205.

Since the capsule cavities 302, 303 have the same volume compared to each other, the capsules 302, 303 are crushed at the same time by the impingement surface 401 while applying equal force to each.

Once the outer shell 207 is broken and the property modifying agent 208 is released into the wick 206, the wick 206 may also act a filter. That is, the wick 206 prevents any fragments of outer shell 207 caused by the rupturing of the capsule 204, 205 from being inhaled by the user.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A smoking substitute apparatus comprising:
a compartment comprising an aerosol former,
a wick downstream of the compartment,
a cavity adjacent to the wick, adapted to receive and hold a frangible capsule comprising a property modifying agent, and
a cap, wherein
the cap is movable between a pre-activated position in which the cavity is adapted to hold an intact frangible capsule, and an activated position in which an impingement surface of the apparatus intrudes into the cavity;
such that, when a frangible capsule is positioned within the cavity, movement of the cap from the pre-activated position to the activated position ruptures the capsule to release the property modifying agent to the wick.

2. The smoking substitute apparatus according to claim 1, wherein the cavity is adapted to receive and hold more than one frangible capsule.

3. The smoking substitute apparatus according to claim 1 or 2, wherein the apparatus comprises a plurality of cavities adjacent to the to the wick, wherein each cavity is adapted to receive and hold a frangible capsule comprising a property modifying agent.

4. The smoking substitute apparatus according to claim 3, wherein the cavities are arranged regularly around the wick.

5. The smoking substitute apparatus according to claim 4, wherein the apparatus has two cavities on opposite sides of the wick.

6. The smoking substitute apparatus according to any of claims 3 to 5, wherein the dimensions of each cavity are the same as one another in the activated position.

7. The smoking substitute apparatus according to any of claims 3 to 5, wherein the dimensions of each cavity differ from one another in the activated position.

8. The smoking substitute apparatus according to any of claims 3 to 7, wherein the apparatus comprises a frangible capsule containing a flavourant in one cavity and a frangible capsule containing water in another cavity.

9. The smoking substitute apparatus according to any one of claims 1 to 8 wherein the cap is moveable to a second activation position wherein the impingement surface of the apparatus intrudes further into the cavity than in the activated position; such that when more than one frangible capsule is positioned with the same or different cavity, movement of the cap from the pre-activated position to the activated position ruptures a first capsule to release the property modifying agent to the wick and movement from the activated position to the second activated position ruptures a second capsule to release the property modifying agent to the wick.

10. The smoking substitute apparatus according to any of claims 1 to 9, wherein the cavity is defined between the cap and a base of the apparatus to which the cap is fitted, wherein the base and the cap are movable relative to one another causing the impingement surface to intrude into the cavity.

11. The smoking substitute apparatus according to claim 10, wherein the impingement surface forms a part of a base of the apparatus, and moving the cap towards the base causes the impingement surface to intrude into the cavity.

12. The smoking substitute apparatus according to claim 10 or 11, wherein the cap and the base are separate structures associated with one another in a friction fit arrangement.

13. The smoking substitute apparatus according to claim 12, wherein the base and the cap comprise a detent mechanism to temporarily hold the apparatus in the pre-activated position until the user activates the apparatus to move it into the activated position.

14. A smoking substitute apparatus, comprising:
a compartment comprising an aerosol former,
a wick downstream of the compartment,
a cavity adjacent to the wick,
a frangible capsule comprising a property modifying agent, the frangible capsule being located within the cavity, and
a cap, wherein
the cap is movable between a pre-activated position in which the cavity holds the intact frangible capsule, and an activated position in which an impingement surface of the apparatus is advanced further towards the cavity than in the pre-activated position;
such that movement of the cap from the pre-activated position to the activated position ruptures the capsule to release the property modifying agent to the wick.
